# EUROPÄISCHE PATENTSCHRIFT

(11) **EP 2 015 797 B1**
(45) Veröffentlichungstag und Bekanntmachung des Hinweises auf die Patenterteilung: **05.07.2017**
(21) Anmeldenummer: 07724694.0
(22) Anmeldetag: 27.04.2007
(51) Int. Cl.: A61M 1/36, A61M 1/34

(54) **VERFAHREN ZUR FÜLLUNG UND SPÜLUNG EINES BLUTSCHLAUCHSATZES**
METHOD FOR FILLING AND RINSING A SET OF BLOOD LINES
PROCÉDÉ DE REMPLISSAGE ET DE RINÇAGE D'UN ENSEMBLE DE TUYAUX POUR LE SANG

(30) Priorität: 11.05.2006 DE 102006022122
(43) Veröffentlichungstag der Anmeldung: 21.01.2009
(60) Teilanmeldung: 17172400.8
(73) Patentinhaber: Fresenius Medical Care Deutschland GmbH, 61352 Bad Homburg v.d.H. (DE)
(72) Erfinder: FISCHER, Max, 60323 Frankfurt (DE)
(74) Vertreter: Laufhütte, Dieter
(86) Internationale Anmeldenummer: PCT/EP2007/003767
(87) Internationale Veröffentlichungsnummer: WO 2007/131611

(56) Entgegenhaltungen:
- WO-A-96/40320
- WO-A-99/20376
- DE-A1- 4 240 681
- DE-C1- 10 011 208
- US-A- 5 776 345

## Beschreibung

Die Erfindung betrifft ein Verfahren zur Füllung und Spülung eines Blutschlauchsatzes, der ein Pumpsegment für eine Blutpumpe, eine mit einem Eingang eines Dialysators verbundene arterielle Leitung und eine mit einem Ausgang eines Dialysators verbundene venöse Leitung umfasst, über eine Substituatleitung.

Solche Blutschlauchsätze kommen bei extrakorporalen Bluttherapien, z.B. bei der Hämodialyse, zum Einsatz und bilden dabei den extrakorporalen Blutkreislauf. Als arterielle und venöse Leitung eines solchen Blutschlauchsatzes werden üblicherweise steril verpackte Einmalartikel verwendet, welche vor der Behandlung gefüllt und gespült werden müssen. Dieses Füllen und Spülen des Blutschlauchsatzes wird auch Priming genannt und dient zur Vermeidung des Kontaktes des Blutes mit Luft bei der extrakorporalen Therapie. Hierbei wird das als Disposable-System ausgeführte Blutschlauchsystem mit Substituat gefüllt, bevor der Patient zur Behandlung angeschlossen wird, so dass das extrakorporale System nach dem Priming nahezu luftfrei ist. Nach dem Füllen und Spülen des Schlauchsystems werden venöse und arterielle Leitungen üblicherweise miteinander verbunden, so dass das Substituat in diesem Kreislauf aus arterieller Leitung, Dialysator und venöser Leitung zirkulieren kann, bis der Patient an das System angeschlossen wird. Nach dem Anschluss des Patienten wird das Substituat durch das einströmende Blut verdrängt, so dass ein möglicher Kontakt des Blutes mit der Luft auf ein Minimum reduziert wird.

Verfahren zur Füllung und Spülung von Blutschlauchsätzen unter Verwendung von Beuteln mit physiologischer Lösung und entsprechenden Beuteln zum Auffangen der benutzten Lösung sind bekannt. Dabei wird üblicherweise ein Beutel mit Kochsalzlösung mit der arteriellen Leitung verbunden und diese gefüllt. Nach dem Füllen der arteriellen Leitung wird nun die Blutpumpe verwendet, um auch die venöse Leitung zu füllen und zu spülen. Hierzu wird das Pumpsegment des Blutschlauchsatzes, ein Schlauchabschnitt mit bestimmten Eigenschaften, in die Blutpumpe, üblicherweise eine peristaltische Pumpe bzw. eine Rollenpumpe, eingesetzt, so dass die Blutpumpe über das Pumpsegment Kochsalzlösung in die venöse Leitung pumpen kann.

Um den Füll- und Spülvorgang weitestgehend zu automatisieren, wurde in EP 831 945 B1 ein Verfahren vorgeschlagen, bei welchem ein T-Konnektor zum Einsatz kommt, um zum Füllen und Spülen die arterielle Leitung und die venöse Leitung kurzzuschließen und über den T-Konnektor mit einem Beutel zum Auffangen der benutzten Lösung zu verbinden. Des weiteren sind an den Blutleitungen, das heißt der arteriellen und der venösen Leitung, in der Nähe des Konnektors zwingend Ventile vorgesehen, um den Füll- und Spülvorgang zu steuern. Zuerst wird hierbei das Substituat durch die Verwendung der Schwerkraft von einem Beutel in die arterielle Leitung gefüllt. Nach Öffnen und Schließen der entsprechenden Ventile wird daraufhin die Flüssigkeit von der arteriellen Leitung in die venöse Leitung gefüllt. Nach einem weiteren Öffnen und Schließen der entsprechenden Ventile kann dann das Substituat in dem Kreislauf aus arterieller Leitung, Dialysator und venöser Leitung durch die Blutpumpe zirkuliert werden. Das Anbringen der ansteuerbaren Ventile am Blutschlauchsatz ist dabei umständlich und darf nicht vergessen werden, erfordert zusätzlich elektrische und pneumatische Verbindungen und ist anfällig für Bedienungsfehler der Dialyseschwester, z. B. durch Verwechslung oder fehlerhafte Positionierung der Ventile. Zudem ist es nötig, vor Beginn der automatisierbaren Prozedur manuell eine Quelle mit einem sterilen Fluid (z. B. ein Beutel mit steriler Kochsalzlösung) oberhalb der Behandlungsmaschine aufzuhängen, anzustechen und mit dem Blutschlauchsatz zu verbinden, wobei sichergestellt werden muss, dass die Ventile zuvor richtig montiert und geschlossen sind. Zur Automatisierung der Ventilbetätigung müssen die Ventile zudem hard- und softwareseitig angesteuert werden, wobei gleichzeitig sichergestellt werden muss, dass keines der Ventile versagt oder sich beim Anschließen des Lösungsbeutels in einer falschen Verschlussstellung befindet.

WO 99/20367 offenbart einen Blutschlauchsatz mit einer arteriellen Leitung und einer venösen Leitung. Die beiden Leitungen werden zum Primen über einen Verbinder miteinander verbunden, wobei der Verbinder eine Ausgabeleitung aufweist, über die Primingflüssigkeit aus den Leitungen abgeführt werden kann. Ferner weist der Blutschlauchsatz eine Primingleitung auf, die in die arterielle Leitung an einer Position vor der Blutpumpe mündet. Das Füllen und/oder Spülen der arteriellen und venösen Leitung erfolgt gleichzeitig.

DE 100 11 208 C zeigt ein Verfahren zur Füllung und/oder Spülung eines Blutschlauchsatzes, bei dem die arterielle Leitung und die venöse Leitung miteinander und ggf. mit einer Abzugsleitung verbunden werden. Dabei wird in einer ersten Variante die Blutseite durch Flüssigkeitsübertritt durch die Membran von der Dialysatseite aus befüllt. Hierfür wird die Blutseite zunächst bei stehender Blutpumpe und in geschlossenem Zustand teilweise befüllt, dann die venöse Blasenkammer entlüftet und die venöse Blasenkammer befüllt. Dann wird die Blutpumpe gestartet und weiterhin Flüssigkeit über die Membran auf die Blutseite gedrückt. Die Flüssigkeit wird im Blutkreislauf zirkuliert und Luft am arteriellen Blasenfänger abgeschieden. Nach einer weiteren Füllung der venösen Blasenkammer ist die Blutseite gefüllt und durch Betätigen der Blutpumpe wird ein Spülverfahren eingeleitet. Im weiteren Verlauf wird ein Reinfusionsbehälter befüllt, welcher nach der Behandlung zum Ersatz des dem Patienten reinfundierten, im Blutschlauchsatz befindlichen Blutes genutzt wird. In einer zweiten Variante soll die Blutseite über eine Substitutionsleitung mit einer Subsitutionspumpe befüllt werden, wobei die Substitutionsleitung stromaufwärts oder stromabwärts des Dialysators mit dem Blutschlauchsatz in Verbindung stehen kann, wobei die gleichen Abläufe wie bei der ersten Variante zum Füllen und Spülen eingesetzt werden sollen.

Aufgabe der vorliegenden Erfindung ist es, ein Verfahren zur Füllung und Spülung eines Blutschlauchsatzes zur Verfügung zu stellen, welches sicherer in der Anwendung und einfacher, schneller und kostengünstiger durchführbar ist. Weiterhin ist es Aufgabe der vorliegenden Erfindung, eine entsprechende Blutbehandlungsmaschine und ein entsprechendes Blutbehandlungssystem zur Verfügung zu stellen.

Erfindungsgemäß wird die Aufgabe von einem Verfahren zur Füllung und Spülung eines Blutschlauchsatzes gemäß Anspruch 1 gelöst, sowie durch eine Blutbehandlungsmaschine gemäß Anspruch 12 und Blutbehandlungssystem gemäß Anspruch 13.

Das erfindungsgemäße Verfahren umfasst dabei die an sich bekannten Schritte des Verbindens der arteriellen Leitung mit der venösen Leitung sowie des Verbindens dieser beiden Leitungen mit einem Rinse-Port, Verbinden der Substituatleitung mit einem Substituat-Port, Öffnen des Rinse-Ports, Füllen der arteriellen und der venösen Leitung, Spülen der arteriellen und der venösen Leitung, Schließen des Rinse-Ports, Zirkulieren des Substituats im Kreislauf aus arterieller Leitung, Dialysator und venöser Leitung durch die Blutpumpe, wobei es jedoch dadurch gekennzeichnet ist, dass das Füllen und/oder das Spülen der arteriellen und der venösen Leitung gleichzeitig erfolgt, wobei Substituat aus der Substituatleitung zugeführt wird. Natürlich wird vor Verwendung der Blutpumpe das Pumpsegment des Blutschlauchsatzes in diese eingesetzt.

Insbesondere kann durch dieses Verfahren darauf verzichtet werden, Ventile am Blutschlauchsatz anzubringen, welche das Füllen oder Spülen der arteriellen Leitung und das darauf folgende Füllen oder Spülen der venösen Leitung steuern müssten. Die mit dem Anbringen dieser Ventile verbundenen Fehlerquellen bei der Bedienung sind damit ausgeschlossen. Zudem ermöglicht das gleichzeitige Füllen und/oder Spülen der arteriellen und der venösen Leitungen einen schnelleren Füll- und Spülvorgang, da auf unterschiedliche Verfahrensschritte verzichtet werden kann. Zudem ist das Verfahren auch einfacher zu automatisieren und die Zeit, welche eine Dialyseschwester zur Füllung und Spülung des Blutschlauchsatzes benötigt, wird minimiert. Außerdem ist so der Blutschlauchsatz kostengünstig und muss während des Verfahrens nicht geändert werden. Zudem kann das Verfahren komplett an der Behandlungsmaschine durchgeführt werden und noch vor der Konnektion des Patienten vollständig abgeschlossen werden, so dass der Patient in der Regel während der Vorbereitung der Behandlungsmaschine abwesend sein kann.

Die Schnittstellen für den Blutschlauchsatz sind dabei der Substituat-Port, über welchen Substituat in die Substituatleitung eingeführt wird, sowie der Rinse-Port, über welchen benutztes Substituat wieder abgeführt werden kann. Sowohl Substituat-Port als auch Rinse-Port verfügen üblicherweise über Ventile, mit welchen sie zur Anwendung des Verfahrens geschlossen und geöffnet werden können.

Vorteilhafterweise bilden bei dem erfindungsgemäßen Verfahren das Füllen und das Spülen einen kontinuierlichen Vorgang. Hierdurch ergibt sich insbesondere keinerlei Unterbrechung des Flüssigkeitsstroms, womit die sonst gegebene Gefahr, dass durch zeitweiliges Unterbrechen des nachströmenden Flüssigkeitsstroms in Folge von Luftblasenförderung eine Schaumbildung auftritt, vermieden wird. Hierbei werden also nicht nur arterielle und venöse Leitungen gleichzeitig gefüllt bzw. gespült, sondern dieser Füll- und Spülvorgang erfolgt auch kontinuierlich. Neben der eben erwähnten Erhöhung der Sicherheit erlaubt auch dies eine einfachere Automatisierung, da insbesondere auf den Einsatz von Ventilen am Blutschlauchsatz verzichtet werden kann.

Weiterhin vorteilhafterweise erfolgt dabei das Füllen und das Spülen durch den gleichen Vorgang. Hierdurch muss nicht extra zwischen Füll- und Spülvorgang umgeschaltet werden, so dass sich der Steuerungs- bzw. Bedienaufwand für das Verfahren vermindert und die Sicherheit erhöht.

Erfindungsgemäß erfolgt das gleichzeitige Füllen und/oder Spülen der arteriellen und der venösen Leitung dadurch, dass die Blutpumpe im Blutschlauchsatz Substituat befördert, während gleichzeitig Substituat aus der Substituatleitung zugeführt wird. So muss lediglich auf bereits vorhandene Komponenten des Blutschlauchsatzes zurückgegriffen werden, um den Füll- und/oder Spülvorgang zu steuern. Das Substituat aus der Substituatleitung wird dabei in den Blutschlauchsatz zugeführt, während die Blutpumpe dafür sorgt, dass sowohl die arterielle als auch die venöse Leitung mit dem Substituat durchströmt werden. Hierdurch kann auf Ventile verzichtet werden und sowohl die arterielle als auch die venöse Leitung gleichzeitig gefüllt bzw. gespült werden. Dabei wird das Substituat zwischen Blutpumpe und Dialysator zugeführt, wobei die Blutpumpe rückwärts läuft.

Vorteilhafterweise fördert während des Füll- und/oder Spülvorgangs die Blutpumpe eine Menge n an Substituat, wobei der saugseitigen Leitung zur Blutpumpe eine Menge m+n an Substituat aus der Substituatleitung zugeführt wird. Hierdurch pumpt die Blutpumpe einen Teil n des aus der Substituatleitung zugeführten Substituats durch den druckseitigen Teil des Blutschlauchsatzes, während der saugseitige Teil des Blutschlauchsatzes ab der Zuleitung der Substituatleitung von der verbleibenden Menge m des Substituats aus der Substituatleitung direkt durchströmt wird. Bei diesem Verfahren werden also beide Teile des Blutschlauchsatzes gleichzeitig parallel und in gleicher Richtung durchströmt, wobei beide Substituatströme nach dem Füllen des Blutschlauchsatzes am Rinse-Port wieder zusammentreffen und von dort abgeleitet werden.

Auf diese Art und Weise ergibt sich ein einfaches Verfahren, mit welchem der Blutschlauchsatz sicher und schnell befüllt und gespült werden kann, wobei lediglich die Mengen gesteuert werden müssen, welche von der Blutpumpe gefördert werden bzw. welche aus der Substituatleitung zugeführt werden.

Da die Blutpumpe rückwärts läuft und das Pumpsegment für die Blutpumpe in der arteriellen Leitung integriert ist, pumpt die Blutpumpe damit direkt Substituat durch die arterielle Leitung zum Rinse-Port, während das restliche Substituat den Dialysator und die venöse Leitung durchströmt und sich dann im Rinse-Port mit dem direkt von der Blutpumpe gepumpten Substituat vereinigt.

Bei dem erfindungsgemäßen Verfahren ist das Pumpsegment für die Blutpumpe in der arteriellen Leitung angeordnet. Weiterhin wird das Substituat zwischen Blutpumpe und Dialysator zugeführt, insbesondere am Prädilutionszugang des Blutschlauchsatzes. So durchströmt frisch zugegebenes Substituat zumindest teilweise zuerst den Dialysator, wodurch dieser besonders effektiv gereinigt wird.

Da die Blutpumpe rückwärts läuft, wird dadurch die Menge n an Substituat, welche von der Blutpumpe gepumpt wird, durch die arterielle Leitung in Richtung Rinse-Port gefördert. Die Menge m + n an Substituat, welche bei diesem Verfahren aus der Substituatleitung in den Prädilutionszugang des Blutschlauchsatzes zugeführt wird, wird also teilweise von der Blutpumpe in die arterielle Leitung gepumpt, während der Rest durch den Dialysator und die venöse Leitung strömt.

Zwar kann das erfindungsgemäße Verfahren auch unter Verwendung von Beuteln zum Füllen und Spülen und entsprechenden Beuteln zum Auffangen der benutzten Lösung betrieben werden, besonders vorteilhafterweise kommt es aber in einem System mit integrierter Substituataufbereitung zum Einsatz. Der Substituat-Port und der Rinse-Port sind dabei Teil dieses Systems, wobei über den Substituat-Port das frische Substituat bereit gestellt wird, während über den Rinse-Port das benutzte Substituat zur Aufbereitung zurückgeführt wird. Dies bedeutet insbesondere, dass der Blutschlauchsatz in einen Kreislauf integriert ist.

Weiterhin vorteilhafterweise verfügen dabei beide Ports über ein maschinenseitiges Ventil, so dass die Ventile nicht Bestandteil des Blutschlauchsatzes sein müssen. Insbesondere muss bei einem solchen System mit integrierter Substituataufbereitung nicht mehr auf Beutel zurückgegriffen werden, so dass die Kosten für die Beschaffung, Lagerhaltung, Anwendung und Entsorgung dieser Beutel entfallen. Ein besonderer Vorteil des erfindungsgemäßen Verfahrens besteht insbesondere darin, dass bestehende Systeme mit integrierter Substituataufbereitung nicht hardwaremäßig verändert werden müssen, um das erfindungsgemäße Verfahren anzuwenden. Es bedarf dazu lediglich einer neuen Softwareansteuerung, so dass bestehende Systeme auf einfache Art und Weise mit dem neuen Verfahren ausgestattet werden können.

Vorteilhafterweise erfolgt dabei bei dem erfindungsgemäßen Verfahren die Ansteuerung des Rinse-Ports und der Blutpumpe automatisch. Es müssen bei dem erfindungsgemäßen Verfahren lediglich die Verbindungen des Schlauchsatzes am Anfang hergestellt werden, während der gesamte Füll- und Spülvorgang automatisch erfolgt. Hierdurch wird der Dialyseschwester sehr viel Arbeit erspart, wobei gleichzeitig Fehlermöglichkeiten durch falsche Bedienung ausgeschlossen werden.

Weiterhin vorteilhafterweise wird das Substituat über eine Substituatpumpe zugeführt. Dies ermöglicht eine genaue Steuerung der zugegebenen Menge, so dass das Füllen und das Spülen äußerst kontrolliert erfolgen kann.

Vorteilhafterweise erfolgt die Ansteuerung der Substituatpumpe dabei automatisch, so dass auch hier keine manuellen Bedienschritte von Nöten sind, so dass damit verbundene Fehlerquellen ausgeschlossen sind und Zeit gespart werden kann.

Weiterhin vorteilhafterweise ist ein Pumpsegment für die Substituatpumpe dabei in der Substituatleitung integriert. Insbesondere bei einem Anschluß an ein System mit integrierter Substituataufbereitung muss dieses System hierdurch hardwaremäßig nicht verändert werden, da alle zum Betreiben des Verfahren notwendigen Komponenten außerhalb des Systems zur Verfügung stehen.

Vorteilhafterweise erfolgt auch die Ansteuerung der Substituatpumpe automatisch. Hierzu sollte vorteilhafterweise das System mit integrierter Substituataufbereitung mit einer neuen Software ausgestattet werden, welche das erfindungsgemäße Zusammenwirken von Substituat-Port, Rinse-Port, Blutpumpe und Substituatpumpe steuert. Weiterer insbesondere hardwareseitiger Veränderungen bedarf es nicht, so dass bestehende Systeme einfach mit dem neuen Verfahren ausgestattet werden können.

Vorteilhafterweise kommt dabei zum Verbinden der arteriellen Leitung, der venösen Leitung und des Rinse-Port ein T-Konnektor zum Einsatz, so dass diese Verbindung auf einfache und sichere Weise hergestellt werden kann.

Während beim gleichzeitigen Spülen der arteriellen und der venösen Leitung das Substituat über den Rinse-Port abgeführt wird, ist es ebenso möglich, während dem Spülen bzw. dem Zirkulieren des Substituats auch über den Dialysator Substituat abzuführen.

Weiterhin umfasst die vorliegende Erfindung Blutbehandlungsmaschine mit integrierter Substituataufbereitung zur Durchführung eines Verfahrens nach einem der vorangegangenen Ansprüche, mit einem Substituat-Port und einem Rinse-Port, welche als Schnittstelle für den Blutschlauchsatz dienen und über Ventile verfügen, mit welchen sie zur Anwendung des Verfahrens geschlossen und geöffnet werden können, und mit einer Blutpumpe, einer Substituatpumpe und einem Steuerprogramm, wobei das Steuerprogramm das Zusammenwirken von Substituat-Port, Rinse-Port, Blutpumpe und Substituatpumpe automatisch derart steuert, dass die folgenden Schritte ausgeführt werden:
- Öffnen des Rinse-Ports
- Füllen der arteriellen und der venösen Leitung,
- Spülen der arteriellen und der venösen Leitung,
- Schließen des Rinse-Ports,
- Zirkulieren des Substituats im Kreislauf aus arterieller Leitung, Dialysator und venöser Leitung durch die Blutpumpe,
wobei das Füllen und/oder das Spülen der arteriellen und der venösen Leitung gleichzeitig erfolgt, indem die Blutpumpe im Blutschlauchsatz Substituat befördert, während gleichzeitig über die Substituatpumpe Substituat aus der Substituatleitung zugeführt wird, wobei die Blutpumpe rückwärts läuft..

Weiterhin umfasst die vorliegende Erfindung ein extrakorporales Blutbehandlungssystem aus einer solchen Blutbehandlungsmaschine und einem Blutschlauchsatz, der ein Pumpsegment für die Blutpumpe, eine mit einem Eingang eines Dialysators verbundene arterielle Leitung und eine mit einem Ausgang eines Dialysators verbundene venöse Leitung, eine Substituatleitung und einen Konnektor umfasst, wobei der Konnektor mit der arteriellen Leitung, der venösen Leitung und dem Rinse-Port verbindbar ist, wobei ein Pumpsegment für eine Substituatpumpe in der Substituatleitung integriert ist und wobei die Substituatleitung am Prädilutionszugang zwischen Blutpumpe und Dialysator angeschlossen ist.

Ausführungsbeispiele der vorliegenden Erfindung werden nun anhand von Zeichnungen näher beschrieben. Dabei zeigen:
- Figur 1:: ein Ausführungsbeispiel des erfindungsgemäßen Füll- und Spülvorgangs, und
- Figur 2:: ein Ausführungsbeispiel des erfindungsgemäßen Zirkulierens.

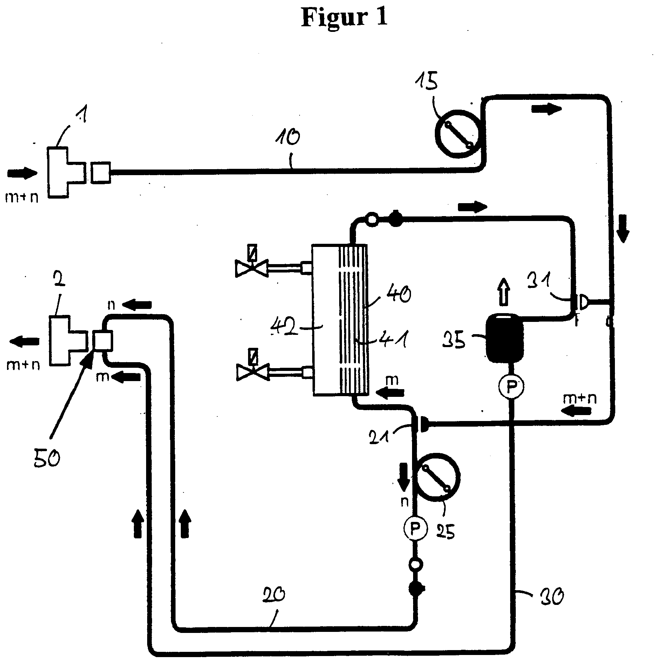

In Figur 1 ist ein Ausführungsbeispiel der Erfindung gezeigt. Das extrakorporale Blutbehandlungssystem mit integrierter Substituataufbereitung umfasst dabei maschinenseitig den Substituat-Port 1 und den Rinse-Port 2. Über den Substituat-Port 1 wird frisches Substituat in den Blutschlauchsatz abgegeben, während über den Rinse-Port 2 gebrauchtes Substituat wieder abgeführt und aufbereitet wird.

Der eigentliche Blutschlauchsatz besteht aus einer arteriellen Leitung 20 und einer venösen Leitung 30. In der arteriellen Leitung 20 befindet sich das Pumpsegment für die Blutpumpe 25, in der venösen Leitung 30 der Luftabscheider 35. Der Dialysator 40 umfasst zwei Kammern, eine Dialysatkammer 42 für die Spülflüssigkeit und eine Blutkammer 41, über welche der Dialysator 40 in den extrakorporalen Blutkreislauf integriert wird. Hierzu ist die arterielle Leitung 20 hinter der Blutpumpe 25 mit dem Eingang der Blutkammer 41 des Dialysators 40 verbunden. Der Ausgang der Blutkammer 41 ist mit der venösen Leitung 30 verbunden, wobei aus der Blutkammer 41 strömende Flüssigkeit zuerst in den Luftabscheider 35 gelangt, um eventuell auftretende Luftblasen abzuscheiden. Zwischen der Blutpumpe 25 und dem Eingang der Blutkammer 41 befindet sich in der arteriellen Leitung 20 der Prädilutionszugang 21. Zwischen dem Ausgang der Blutkammer 41 und dem Luftabscheider 35 befindet sich in der venösen Leitung 30 der Postdilutionszugang, der jedoch bei den vorliegenden Ausführungsbeispielen des erfindungsgemäßen Verfahrens nicht zum Einsatz kommt. Die Substituatleitung 10 ist am Prädilutionszugang angeschlossen und verfügt über ein Pumpsegment für eine Substituatpumpe 15.

Zur Durchführung des erfindungsgemäßen Verfahrens zur Füllung und Spülung des Blutschlauchsatzes ist die Substituatleitung 10 am Substituat-Port 1 des Blutbehandlungssystems angeschlossen. Die patientenseitigen Enden der venösen Leitung 30 und der arteriellen Leitung 20 sind über einen T-Konnektor 50 kurzgeschlossen und mit dem Rinse-Port 2 des Blutbehandlungssystems verbunden. Sowohl Substituat-Port 1 als auch Rinse-Port 2 sind geöffnet.

Bei dem in Figur 1 gezeigten Ausführungsbeispiel wird eine Menge m + n Substituat mittels der Substituatpumpe 15 über den Substituat-Port 1 angesaugt und über den Prädilutionszugang 21 in die arterielle Leitung 20 zwischen Blutpumpe 25 und Eingang des Dialysators 40 eingespeist. Die Blutpumpe 25 läuft in umgekehrter Drehrichtung und pumpt eine Menge n an Substituat direkt durch die arterielle Leitung 20 zum T-Konnektor 50 und damit in den Rinse-Port 2. Der verbleibende Teil des Substituats, also die Menge m, durchströmt zwangsläufig die Blutkammer 41 des Dialysators 40 und danach den Luftabscheider 35, bevor dieser Teilstrom durch die venöse Leitung 30 ebenfalls in den T-Konnektor 50 und damit in den Rinse-Port 2 strömt.

Bei diesem Ausführungsbeispiel werden die arterielle Leitung und die venöse Leitung beim Spülen also parallel und gleichzeitig von zwei Teilströmen durchströmt. Die Teilströme trennen sich beim Prädilutionszugang 21 und vereinigen sich wieder im Rinse-Port, von wo aus sie der Substituataufbereitung des Blutbehandlungssystems zugeführt werden. Auf diese Weise können sowohl venöse als auch arterielle Leitungen gleichzeitig gefüllt werden, wobei zur Steuerung dieses Vorgangs lediglich die Blutpumpe 25 und die Substituatpumpe 15 angesteuert werden müssen, ohne dass am Blutschlauchsatz weitere Ventile vorgesehen sein müssen. Auch geschehen so Füll- und Spülvorgang in einem einzigen Arbeitsgang, da das Substituat aus dem Substituat-Port 1 zuerst den Blutschlauchsatz füllt und dann bei weiter laufenden Pumpen durchspült, wobei das gebrauchte Substituat über den geöffneten Rinse-Port 2 abfließt.

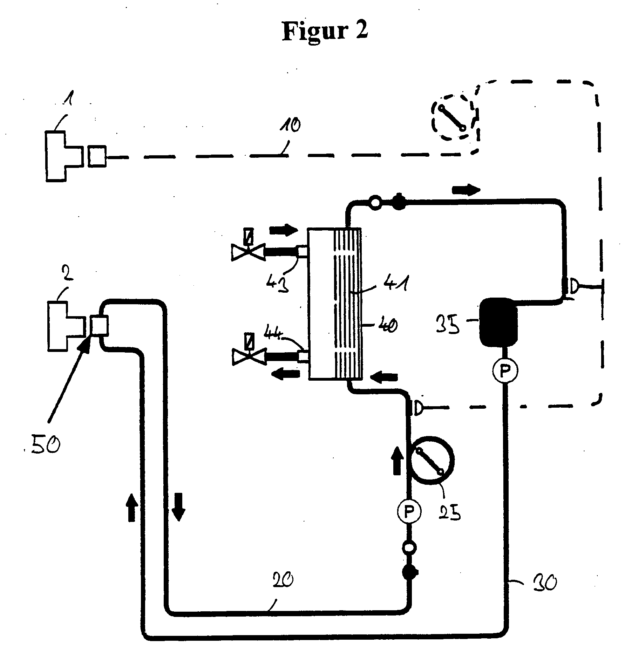

In Figur 2 wurden nun sowohl Substituat-Port 1 als auch Rinse-Port 2 geschlossen, so dass kein Substituat mehr in das Blutschlauchsystem zufließt und auch kein Substituat mehr abfließt. In diesem Vorzirkulierbetrieb arbeitet die Blutpumpe 25 nun in ihrer üblichen Drehrichtung und fördert somit vorwärts. Das Substituat zirkuliert im Blutschlauchsatz nun im Kreislauf in der Reihenfolge Dialysator 40, Luftabscheider 35, venöse Patientenleitung 30, T-Konnektor 50, arterielle Patientenleitung 20, bevor es wieder in die Blutpumpe 25 gelangt und von dort aus weiter gepumpt wird. Damit ist auch der Übergang vom Spülbetrieb in den Vorzirkulierbetrieb möglich, ohne dass Anschlüsse geändert werden müssen oder Ventile am Blutschlauchsatz geöffnet oder geschlossen werden müssen. Wiederum ist also die Steuerung allein über das Öffnen und Schließen der maschinenseitigen Ports und die Ansteuerung der Pumpen möglich.

Zum Beenden des Vorzirkulierens wird die Blutpumpe 25 angehalten. Dann werden die arterielle Leitung 20 sowie die venöse Leitung 30 vom T-Konnektor 50 getrennt, so dass der Patient an diese Leitungen konnektiert werden kann.

Die Änderungen zum Einsatz des erfindungsgemäßen Verfahrens zur Füllung und Spülung des Blutschlauchsatzes, welche an bestehenden Blutbehandlungssystemen vorgenommen werden müssen, beschränken sich dabei auf den Einsatz des speziellen T-Konnektors 50, der als kostengünstiger Einmalartikel ausgeführt werden kann, und der integrierten Substituatpumpe, sowie die Änderung des Steuerprogramms der Behandlungsmaschine, wobei keine neuen Komponenten berücksichtigt bzw. angesteuert werden müssen. Die Änderungen beschränken sich deshalb auf Änderungen der Software. Insbesondere ist die Notwendigkeit einer zusätzlichen, störenden und mit Kosten verbundenen elektrischen Verkabelung nicht gegeben.

Weiterhin ergibt sich der Vorteil, dass der Blutschlauchsatz komplett maschinengesteuert und kontrolliert befüllt und gespült wird und zudem ohne Unterbrechung zum Vorzirkulieren des Substituats übergegangen wird. Insbesondere erfolgt die Füllung und Spülung dabei kontinuierlich und ohne jede Unterbrechung des Flüssigkeitsstroms, was die Sicherheit zusätzlich erhöht. Die mit manuellen Behandlungsschritten verbundenen Fehlerquellen sind damit ausgeschlossen.

## Patentansprüche

1. Verfahren zur Füllung und Spülung eines Blutschlauchsatzes, der ein Pumpsegment für eine Blutpumpe, eine mit einem Eingang eines Dialysators (40) verbundene arterielle Leitung (20) und eine mit einem Ausgang eines Dialysators (40) verbundene venöse Leitung (30) umfasst, über eine Substituatleitung (10), wobei das Verfahren die Schritte umfasst:
- Verbinden der arteriellen Leitung (20) mit der venösen Leitung (30) sowie Verbinden dieser beiden Leitungen mit einem Rinse-Port (2),
- Verbinden der Substituatleitung (10) mit einem Substituat-Port (1),
- Öffnen des Rinse-Ports (2)
- Füllen der arteriellen (20) und der venösen Leitung (30),
- Spülen der arteriellen (20) und der venösen Leitung (30),
- Schließen des Rinse-Ports (2),
- Zirkulieren des Substituats im Kreislauf aus arterieller Leitung (20), Dialysator (40) und venöser Leitung (30) durch die Blutpumpe (25),
wobei die Blutpumpe (25) in der arteriellen Leitung (20) angeordnet ist und das Füllen und/oder das Spülen der arteriellen (20) und der venösen Leitung (30) gleichzeitig erfolgt,
wobei das gleichzeitige Füllen und/oder Spülen der arteriellen (20) und der venösen Leitung (30) dadurch erfolgt, dass die Blutpumpe (25) im Blutschlauchsatz Substituat befördert, während gleichzeitig über eine Substituatpumpe Substituat aus der Substituatleitung (10) zugeführt wird, wobei das Substituat zwischen Blutpumpe (25) und Dialysator (40) zugeführt wird und wobei die Blutpumpe (25) rückwärts läuft.

2. Verfahren nach Anspruch 1, wobei das Füllen und das Spülen einen kontinuierlichen Vorgang darstellen.

3. Verfahren nach Anspruch 1, wobei das Füllen und das Spülen durch den gleichen Vorgang erfolgen.

4. Verfahren nach Anspruch 1, wobei die Blutpumpe (25) während des Füll- und/oder Spülvorgangs eine Menge n an Substituat befördert und der saugseitigen Leitung der Blutpumpe (25) eine Menge m + n an Substituat aus der Substituatleitung (10) zugeführt wird.

5. Verfahren nach Anspruch 1, wobei Substituat-Port (1) und Rinse-Port (2) Teil eines Systems mit integrierter Substituataufbereitung sind.

6. Verfahren nach Anspruch 1, wobei die Ansteuerung des Rinse-Ports (2) und der Blutpumpe (25) automatisch erfolgt.

7. Verfahren nach Anspruch 1, wobei ein Pumpsegment für eine Substituatpumpe (25) in der Substituatleitung integriert ist.

8. Verfahren nach Anspruch 1, wobei die Ansteuerung der Substituatpumpe (15) automatisch erfolgt.

9. Verfahren nach Anspruch 1, wobei die arterielle Leitung (20), die venöse Leitung (30) und der Rinse-Port (2) über einen T-Konnektor (50) verbunden werden.

10. Verfahren nach Anspruch 1, wobei beim gleichzeitigen Spülen der arteriellen (20) und der venösen Leitung (30) Substituat über den Rinse-Port (2) abgeführt wird.

11. Verfahren nach Anspruch 1, wobei beim gleichzeitigen Spülen der arteriellen (20) und der venösen Leitung (30) oder beim Zirkulieren Substituat über den Dialysator (40) abgeführt wird.

12. Blutbehandlungsmaschine mit integrierter Substituataufbereitung zur Durchführung eines Verfahrens nach einem der vorangegangenen Ansprüche, mit einem Substituat-Port (1) und einem Rinse-Port (2), welche als Schnittstelle für den Blutschlauchsatz dienen und über Ventile verfügen, mit welchen sie zur Anwendung des Verfahrens geschlossen und geöffnet werden können, und mit einer Blutpumpe (25), einer Substituatpumpe und einem Steuerprogramm, wobei das Steuerprogramm das Zusammenwirken von Substituat-Port, Rinse-Port, Blutpumpe und Substituatpumpe automatisch derart steuert, dass die folgenden Schritte ausgeführt werden:
- Öffnen des Rinse-Ports (2)
- Füllen der arteriellen (20) und der venösen Leitung (30),
- Spülen der arteriellen (20) und der venösen Leitung (30),
- Schließen des Rinse-Ports (2),
- Zirkulieren des Substituats im Kreislauf aus arterieller Leitung (20), Dialysator (40) und venöser Leitung (30) durch die Blutpumpe (25),
wobei das Füllen und/oder das Spülen der arteriellen (20) und der venösen Leitung (30) gleichzeitig erfolgt, indem die Blutpumpe (25) im Blutschlauchsatz Substituat befördert, während gleichzeitig über die Substituatpumpe Substituat aus der Substituatleitung (10) zugeführt wird, wobei die Blutpumpe (25) rückwärts läuft.

13. Extrakorporales Blutbehandlungssystem aus einer Blutbehandlungsmaschine gemäß Anspruch 12 und einem Blutschlauchsatz, der ein Pumpsegment für die Blutpumpe, eine mit einem Eingang eines Dialysators (40) verbundene arterielle Leitung (20) und eine mit einem Ausgang eines Dialysators (40) verbundene venöse Leitung (30), eine Substituatleitung und einen Konnektor umfasst, wobei der Konnektor mit der arteriellen Leitung, der venösen Leitung und dem Rinse-Port verbindbar ist, wobei ein Pumpsegment für eine Substituatpumpe in der Substituatleitung integriert ist und wobei die Substituatleitung (10) am Prädilutionszugang (21) zwischen Blutpumpe (25) und Dialysator (40) angeschlossen ist.

## Claims

1. A method for the filling and flushing of a blood tubing set, comprising a pump segment for a blood pump, an arterial line (20) connected to an inlet of a dialyzer (40) and a venous line (30) connected to an outlet of a dialyzer (40), via a substituate line (10), the method comprising the following steps:
- connecting the arterial line (20) to the venous line (30) and connecting these two lines to a rinse port (2);
- connecting the substituate line (10) to a substituate port (1);
- opening the rinse port (2);
- filling the arterial line (20) and the venous line (30);
- flushing the arterial line (20) and the venous line (30);
- closing the rinse port (2);
- circulating the substituate in the circuit of arterial line (20), dialyzer (40) and venous line (30) by the blood pump (25),
wherein the blood pump (25) is arranged in the arterial line (20) and the filling and/or the flushing of the arterial line (20) and the venous line (30) takes/take place simultaneously,
wherein the simultaneous filling and/or flushing of the arterial line (20) and of the venous line (30) takes place in that the blood pump (25) transports substituate in the blood tubing set, while substituate is simultaneously supplied from the substituate line (10) via a substituate pump, with the substituate being supplied between the blood pump (25) and the dialyzer (40) and with the blood pump (25) running backwards.

2. A method in accordance with claim 1, wherein the filling and the flushing represent a continuous process.

3. A method in accordance with claim 1, wherein the filling and the flushing take place by the same process.

4. A method in accordance with claim 1, wherein the blood pump (25) transports an amount n of substituate during the filling and/or flushing process and an amount m + n of substituate is supplied from the substituate line (10) to the suction-side line of the blood pump (25).

5. A method in accordance with claim 1, wherein the substituate port (1) and the rinse port (2) form part of a system with substituate preparation.

6. A method in accordance with claim 1, wherein the control of the rinse port (2) and of the blood pump (25) takes place automatically.

7. A method in accordance with claim 1, wherein a pump segment for a substituate pump (25) is integrated in the substituate line.

8. A method in accordance with claim 1, wherein the control of the substituate pump (15) takes place automatically.

9. A method in accordance with claim 1, wherein the arterial line (20), the venous line (30) and the rinse port (2) are connected via a T connector (50).

10. A method in accordance with claim 1, wherein substituate is drained off via the rinse port (2) with the simultaneous flushing of the arterial line (20) and of the venous line (30).

11. A method in accordance with claim 1, wherein substituate is drained off via the dialyzer (40) with the simultaneous flushing of the arterial line (20) and of the venous line (30) or during circulation.

12. A blood treatment machine with integrated substituate preparation for carrying out a method in accordance with any one of the preceding claims, having a substituate port (1) and a rinse port (2) which serve as an interface for the blood tubing set and have valves via which they can be opened and closed for the application of the method, und having a blood pump (25), a substituate pump and a control program, with the control program automatically controlling the cooperation between substituate port, rinse port, blood pump and substituate pump such that the following steps are carried out:
- Opening the rinse port (2)
- Filling the arterial line (20) and the venous line (30)
- Flushing the arterial line (20) and the venous line (30)
- Closing the rinse port (2)
- Circulating the substituate in the circuit of arterial line (20), dialyzer (40) and venous line (30) by the blood pump (25),
with the filling and/or flushing of the arterial line (20) and the venous line (30) taking place simultaneously in that the blood pump (25) transports substituate in the blood tubing set, with substituate simultaneously being supplied from the substituate line (10) via the substituate pump, with the blood pump (25) running backwards.

13. An extracorporeal blood treatment system consisting of a blood treatment machine in accordance with claim 12 and a blood tubing set comprising a pump segment for the blood pump, an arterial line (20) connected to an inlet of a dialyzer (40) and a venous line (30) connected to an outlet of a dialyzer (40), a substituate line and a connector, with the connector being connectable to the arterial line, the venous line and the rinse port, with a pump segment for a substituate pump being integrated in the substituate line and with the substituate line (10) being connected to the pre-dilution port (21) between blood pump (25) and dialyzer (40).

## Revendications

1. Procédé de remplissage et de rinçage d'un ensemble de tuyaux pour le sang, qui comprend un segment de pompe pour une pompe à sang, une ligne artérielle (20) reliée à une entrée d'un dialyseur (40) et une ligne veineuse (30) reliée à une sortie d'un dialyseur (40), par le biais d'une ligne de liquide de substitution (10), le procédé comprenant les étapes consistant à :
- relier la ligne artérielle (20) à la ligne veineuse (30) ainsi que relier ces deux lignes à un port de rinçage (2),
- relier la ligne de liquide de substitution (10) à un port de liquide de substitution (1),
- ouvrir le port de rinçage (2)
- remplir la ligne artérielle (20) et la ligne veineuse (30),
- rincer la ligne artérielle (20) et la ligne veineuse (30),
- fermer le port de rinçage (2),
- faire circuler le liquide de substitution dans le circuit formé par la ligne artérielle (20), le dialyseur (40) et la ligne veineuse (30) au moyen de la pompe à sang (25),
dans lequel la pompe à sang (25) est disposée dans la ligne artérielle (20) et le remplissage et/ou le rinçage de la ligne artérielle (20) et la ligne veineuse (30) est/sont effectué(s) simultanément,
dans lequel le remplissage et/ou rinçage simultané(s) de la ligne artérielle (20) et la ligne veineuse (30) est/sont effectué(s) par le fait que la pompe à sang (25) transporte du liquide de substitution dans l'ensemble de tuyaux pour le sang, pendant qu'en même temps du liquide de substitution est alimenté depuis la ligne de liquide de substitution (10) par le biais d'une pompe à liquide de substitution, le liquide de substitution étant alimenté entre la pompe à sang (25) et le dialyseur (40) et la pompe à sang (25) fonctionnant en arrière.

2. Procédé selon la revendication 1, dans lequel le remplissage et le rinçage constituent un processus continu.

3. Procédé selon la revendication 1, dans lequel le remplissage et le rinçage sont effectués par le même processus.

4. Procédé selon la revendication 1, dans lequel la pompe à sang (25) transporte une quantité n de liquide de substitution pendant le processus de remplissage et/ou de rinçage et une quantité m + n de liquide de substitution est alimentée depuis la ligne de liquide de substitution (10) dans la ligne côté aspiration de la pompe à sang (25).

5. Procédé selon la revendication 1, dans lequel le port de liquide de substitution (1) et le port de rinçage (2) font partie d'un système avec traitement intégré du liquide de substitution.

6. Procédé selon la revendication 1, dans lequel la commande du port de rinçage (2) et de la pompe à sang (25) est effectuée automatiquement.

7. Procédé selon la revendication 1, dans lequel un segment de pompe pour une pompe à liquide de substitution (25) est intégré dans la ligne de liquide de substitution.

8. Procédé selon la revendication 1, dans lequel la commande de la pompe à liquide de substitution (15) est effectuée automatiquement.

9. Procédé selon la revendication 1, dans lequel la ligne artérielle (20), la ligne veineuse (30) et le port de rinçage (2) sont reliés par le biais d'un connecteur en T (50).

10. Procédé selon la revendication 1, dans lequel, lors du rinçage simultané de la ligne artérielle (20) et la ligne veineuse (30), du liquide de substitution est évacué par le biais du port de rinçage (2).

11. Procédé selon la revendication 1, dans lequel, lors du rinçage simultané de la ligne artérielle (20) et la ligne veineuse (30) ou lors de la circulation, du liquide de substitution est évacué par le biais du dialyseur (40).

12. Machine de traitement du sang avec traitement de liquide de substitution intégré, destinée à exécuter un procédé selon l'une des revendications précédentes, comprenant un port de liquide de substitution (1) et un port de rinçage (2), qui servent d'interface pour l'ensemble de tuyaux pour le sang et qui disposent de vannes, au moyen desquelles ils peuvent être fermés et ouverts pour l'application du procédé, et comprenant une pompe à sang (25), une pompe à liquide de substitution et un programme de commande, le programme de commande commandant automatiquement l'action conjointe du port de liquide de substitution, du port de rinçage, de la pompe à sang et de la pompe à liquide de substitution de telle manière que les étapes suivantes sont exécutées :
- ouvrir le port de rinçage (2)
- remplir la ligne artérielle (20) et la ligne veineuse (30),
- rincer la ligne artérielle (20) et la ligne veineuse (30),
- fermer le port de rinçage (2),
- faire circuler le liquide de substitution dans le circuit formé par la ligne artérielle (20), le dialyseur (40) et la ligne veineuse (30) au moyen de la pompe à sang (25),
dans lequel le remplissage et/ou le rinçage de la ligne artérielle (20) et la ligne veineuse (30) est/sont effectué(s) simultanément par le fait que la pompe à sang (25) transporte du liquide de substitution dans l'ensemble de tuyaux pour le sang, pendant qu'en même temps du liquide de substitution est alimenté depuis la ligne de liquide de substitution (10) par le biais de la pompe à liquide de substitution, la pompe à sang (25) fonctionnant en arrière.

13. Système de traitement du sang extracorporel constitué d'une machine de traitement du sang selon la revendication 12 et d'un ensemble de tuyaux pour le sang, qui comprend un segment de pompe pour la pompe à sang, une ligne artérielle (20) reliée à une entrée d'un dialyseur (40) et une ligne veineuse (30) reliée à une sortie d'un dialyseur (40), une ligne de liquide de substitution et un connecteur, le connecteur pouvant être relié à la ligne artérielle, la ligne veineuse et le port de rinçage, un segment de pompe pour une pompe à liquide de substitution étant intégré dans la ligne de liquide de substitution et la ligne de substitution (10) étant raccordée à l'arrivée de prédilution (21) entre la pompe à sang (25) et le dialyseur (40).
